**Europäisches Patentamt**

**European Patent Office**

**Office européen des brevets**

(19)

(11) Veröffentlichungsnummer: **0 122 879**
**B1**

(12) # EUROPÄISCHE PATENTSCHRIFT

(45) Veröffentlichungstag der Patentschrift:
27.08.86

(21) Anmeldenummer: 84810160.6

(22) Anmeldetag: 02.04.84

(51) Int. Cl.⁴: **C 07 C 15/20**, C 07 C 1/253

(54) **Verfahren zur Herstellung von Perylen.**

(30) Priorität: 08.04.83  CH 1898/83

(43) Veröffentlichungstag der Anmeldung:
24.10.84 Patentblatt 84/43

(45) Bekanntmachung des Hinweises auf die Patenterteilung:
27.08.86 Patentblatt 86/35

(84) Benannte Vertragsstaaten:
**CH DE FR GB IT LI NL**

(56) Entgegenhaltungen:
**DE - C - 486 491**

(73) Patentinhaber: **CIBA-GEIGY AG, Klybeckstrasse 141,
CH-4002 Basel (CH)**

(72) Erfinder: **Bäbler, Fridolin, Dr., Route du couchant 12,
CH-1723 Marly (CH)**

ACTORUM AG

## Beschreibung

Die Erfindung betrifft ein Verfahren zur Herstellung von Perylen durch Decarboxylierung von Perylentetracarbonsäure oder deren Derivaten.

Perylen kann nach verschiedenen Methoden hergestellt werden, wobei die meisten Verfahren jedoch nur geringe Ausbeuten ergeben.

So erhielten beispielsweise G.M. Badger et al. Perylen durch Dehydrocyclisierung von Naphthalin mit $AlCl_3$ als Katalysator in einer Ausbeute von 1% (Journ. of the Chem. Soc. [1957], 4417) und R. Scholl et al. ausgehend von 1,1'-Binaphthyl in einer Ausbeute von 10% (Chem. Ber. 43, [1910] 2202).

In der DE-PS 486 491 wird ein Verfahren beschrieben, wonach Perylen in guten Ausbeuten durch Decarboxylierung von Perylencarbonsäuren, deren Salzen und Derivaten in 12%iger wässriger Kalilauge im Autoklaven beim Erwärmen auf 250° während mehreren Stunden erhalten wird. Das auf diese Art hergestellte Perylen ist jedoch stark verunreinigt und muss einem nachträglichen Reinigungsprozess unterworfen werden. Ein weiterer Nachteil dieses Verfahrens ist die Notwendigkeit der Verwendung spezieller Autoklaven mit hohen Anschaffungskosten.

Nach der JP-OS 71/21863 wird Perylen durch Thermolyse von Perylentetracarbonsäure oder deren Derivaten in Gegenwart von Natronkalk bei ca. 440° unter Vakuum erhalten. Das nach diesem Verfahren erhaltene Perylen muss infolge mangelnder Reinheit ebenfalls nachträglich umkristallisiert und über Aluminiumoxid gereinigt werden. Die Vakuumsublimation hat ausserdem den Nachteil, dass Verunreinigungen mitgerissen werden können. Bei Temperaturen von 400-500° und Drücken von $10^{-5}$-$10^{-11}$ bar sind zudem spezielle Apparaturen notwendig.

Gegenstand der Erfindung ist somit ein Verfahren zur Herstellung von Perylen durch Erhitzen von Perylentetracarbonsäure, deren Anhydriden oder Salzen in An- oder Abwesenheit von Metalloxiden, -hydroxiden oder -carbonaten auf Temperaturen zwischen 380-550°C, bevorzugt zwischen 400-460°C, wobei man bei Normaldruck oder nur schwach vermindertem Druck arbeitet und das entstehende Perylen mit einem Inertgasstrom aus dem Reaktionsgefäss entfernt.

Als Ausgangsstoffe eignen sich Perylen-mono-, di-, tri- oder Tetracarbonsäuren, sowie deren Salze, beispielsweise die Na-, K-, Ca- oder Mg-Salze. Bevorzugtes Ausgangsprodukt ist jedoch das Perylentetracarbonsäuredianhydrid.

Als Metallverbindungen, welche die Bildung des Perylens begünstigen kommen beispielsweise Erdalkalioxide und ZnO, deren Carbonate und Hydroxide, sowie Aluminiumoxid und -hydroxid einzeln oder in Mischung in Betracht. Besonders geeignet sind Alkali- und Erdalkalihydroxide, Erdalkalioxide, einzeln oder in Mischung, beispielsweise Natronkalk, da diese die Bildung von Perylen bei Temperaturen unter 480°C ermöglichen.

Man verwendet auf einen Teil der Perylencarbonsäure oder ihres Derivates zweckmässig 1-6, vorzugsweise 1,5-3,5 Teile der Metallverbindung. Die Reaktionsteilnehmer werden vorteilhaft vor dem Erhitzen innig vermischt. Dies geschieht beispielsweise in Mischern oder durch Vermahlen, beispielsweise in Kugelmühlen, Perlmühlen oder anderen Mahlaggregaten.

Die bevorzugt verwendeten Erdalkalioxide können in trockenem Zustand in einem Intensivmischer mit der Perylentetracarbonsäure oder deren Derivaten vermischt werden. Magnesiumoxid wird zweckmässig mit dem Perylentetracarbonsäuredianhydrid in Wasser zusammen vermahlen. Nach Entfernung des Wassers erhält man eine innige Mischung von MgO, $Mg(OH)_2$, Mg-Salz und Anhydrid der Perylentetracarbonsäure.

Das Erhitzen der Ausgangsstoffe erfolgt in einem Inertgasstrom. Als Inertgas wird aus wirtschaftlichen Gründen in erster Linie Stickstoff verwendet. Selbstverständlich können auch Edelgase eingesetzt werden. Zweckmässig wird bei Drucken von mehr als 0,7 bar (1 bar = $10^6$ dyne pro $cm^2$), bevorzugt bei Atmosphärendruck gearbeitet.

Die Reaktionsdauer beträgt je nach Art der verwendeten Metallverbindung zwischen einer und mehreren Stunden. Das gebildete Perylen wird zweckmässig in gasförmigem Zustand vom Inertgasstrom in ein ausserhalb der Heizzone befindliches Auffanggefäss geleitet. Unmittelbar beim Austritt aus der Heizzone wandelt sich das Perylen vom gasförmigen in den festen Zustand um und wird als gelbes Pulver im Auffanggefäss niedergeschlagen. Je nach Geschwindigkeit des Inertgasstromes fällt das Perylen in feiner bis gröber kristalliner Form an. Die Anwesenheit von Wasser, herrührend von den Metallhydroxiden, in der Aufheizphase und am Anfang der Reaktion stört die Bildung des Perylens nicht.

Das verfahrengemäss erhaltene Perylen weist eine hohe Reinheit auf und braucht weder nachträglich sublimiert noch in organischen Lösungsmitteln umkristallisiert zu werden.

Es ist überraschend, dass nach dem erfindungsgemässen Verfahren bei Normaldruck und Temperaturen von 460° und darunter Perylen in guten Ausbeuten und sehr hoher Reinheit erhalten werden kann.

Perylen ist ein sehr stabiler polyzyklischer aromatischer Kohlenwasserstoff, der in neuerer Zeit aufgrund der besonderen physikalischen Eigenschaften stark an Bedeutung gewonnen hat. Dabei ist eine hohe Reinheit des Perylens von entscheidender Bedeutung.

Von den vielen Applikationsmöglichkeiten von Perylen seien u.a. folgende erwähnt:
Die Verwendung von Perylen zur Herstellung
— von elektrophoretischen Tonern
  (JP-Anm. 82-222,247; Prior. 15.7.1980)
— von blau elektroluminiszierenden Filmen
  (P.S. Vincett et al., Thin Solid Films 94 [1982] 171-183)
— von photoempfindlichen Systemen
  (US-PS 3.729.313, Prior. 6.12.71)
— von elektrisch hochleitenden Verbindungen
  (Koch P. et al., Mol. Crist. Liq. Crist. 86 [1-4] [1982] 1827-41)
— von Charge-Transfer-Komplexverbindungen, beispielsweise mit Jod (Cobb, Wallis, J. phys. Chem. 72, [1968], 2968, 2992) oder mit 1,3,5-Trinitrobenzol (Orchin, Friedel, J. Amer. Chem. Soc. 68 [1946] 573).

In den nachfolgenden Beispielen bedeuten die Teile, sofern nichts anderes angegeben, Gewichtsteile und die Grade Celsiusgrade.

*Beispiel 1*

52 Teile Magnesiumoxid und 28 Teile Perylentetracarbonsäuredianhydrid werden in einer Glasperlmühle in 550 Teilen Wasser suspendiert und mit 1900 Teilen Glaskugeln (Durchmesser 4,5-5,0 cm) bei einer Rührgeschwindigkeit von 320 U/min unter Aussenkühlung mit Wasser während 4 1/2 Stunden intensiv gemahlen. Dann werden die Glaskugeln von der Suspension abgetrennt und mit Wasser etwas nachgewaschen. Die Suspension wird abfiltriert und der Presskuchen ohne Waschen im Vakuumschrank bei 80-90°C getrocknet und pulverisiert.

Auf diese Art erhält man 91 Teile einer Mischung, welche aus MgO, Mg(OH)$_2$, dem Mg-Salz und Anhydrid der Perylentetracarbonsäure besteht. Eine Analyse ergab einen Perylentetracarbonsäuredianhydridanteil von ca. 27%.

28,5 Teile der oben hergestellten Mischung werden in einem Glasgefäss unter ständigem Stickstoffstrom auf 460° erwärmt. Der Stickstoff wird aus dem Reaktionsgefäss durch ein Auffanggefäss geleitet, welches sich ausserhalb der Heizzone befindet. Sobald sich das Reaktionsgemisch auf etwa 150° erwärmt hat, entsteht Wasserdampf, der vom Inertgas mitgerissen wird. Nach ca. 10 Minuten bei 460° entweicht mit den Inertgas neben Wasser und CO$_2$ Perylen, welches sich in gelber fester Form im Auffanggefäss niederschlägt. Nach weiteren 45 Minuten wird nur noch Perylen vom Inertgas aus dem Reaktionsgefäss befördert. Man lässt solange bei 460°C Stickstoff durch das Reaktionsgefäss leiten, bis kein Perylen mehr entweicht. Das aufgefangene Perylen wird im Vakuumtrockenschrank von noch anhaftendem Wasser bei 80°C befreit. Man erhält 3,2 g gelbes Perylen mit einem Schmelzpunkt von 278° und hoher Reinheit, wie aus spektroskopischen Analysen hervorgeht. Die Ausbeute beträgt 65%, bezogen auf Perylentetracarbonsäuredianhydrid.

*Beispiel 2*

In einem Osterizer Labormischer werden 30 Teile Perylentetracarbonsäuredianhydrid und 100 Teile Natronkalk, [einem Gemisch von NaOH mit Ca-(OH)$_2$], während einigen Minuten gut vermischt. Die Farbe des roten Perylentetracarbonsäuredianhydrids schlägt dabei in olivgrün um. Die Mischung wird in einem Glasgefäss unter ständigem Stickstoffstrom in 1 1/2 Stunden auf 445°C erwärmt. Der Stickstoff wird durch das Reaktionsgefäss und dann durch ein Auffanggefäss, welches sich ausserhalb der Heizzone befindet, geleitet. Während der Aufheizphase entweicht mit dem Inertgasstrom etwas Wasserdampf. Nach ca. 15 Minuten bei 440-445° bildet sich Perylen, welches vom Stickstoff ins Auffanggefäss befördert wird. Man lässt solange bei 440-445°C Stickstoff durch das Reaktionsgefäss leiten, bis kein Perylen mehr entweicht. Das aufgefangene Perylen wird im Vakuumtrockenschrank von noch anhaftenden Spuren von Wasser bei 80°C befreit. Man erhält 15,5 g Perylen, mit einem Schmelzpunkt von 278-279°C. Die Ausbeute beträgt 80% bezogen auf eingesetztes Perylentetracarbonsäuredianhydrid.

Das Absorptionsspektrum des so erhaltenen Perylens weist in Dimethylformamid bei $\lambda_{max}$ 435 nm einen molaren Extinktionskoeffizienten log $\varepsilon$ = 4,532 auf.

*Beispiel 3*

Verfährt man analog wie in Beispiel 2 beschrieben, leitet jedoch Stickstoff unter schwachem Unterdruck durch das Reaktionsgefäss und anschliessend in das sich ausserhalb der Heizzone befindliche Auffanggefäss, so erhält man Perylen in ähnlich guter Ausbeute und Qualität.

**Patentansprüche**

1. Verfahren zur Herstellung von Perylen durch Erhitzen von Perylencarbonsäuren, deren Anhydriden oder Salzen in An- oder Abwesenheit von Metalloxiden, -hydroxiden oder -carbonaten auf Temperaturen zwischen 380-550°C, dadurch gekennzeichnet, dass man bei Normaldruck oder schwach vermindertem Druck arbeitet und das entstehende Perylen mit einem Inertgasstrom aus dem Reaktionsgefäss entfernt.

2. Verfahren gemäss Anspruch 1, dadurch gekennzeichnet, dass man vom Perylentetracarbonsäuredianhydrid ausgeht.

3. Verfahren nach Anspruch 1, dadurch gekennzeichnet, dass man als Metalloxide Aluminiumoxid, Zinkoxid, Alkali- oder Erdalkalimetalloxide einzeln oder in Mischung verwendet.

4. Verfahren nach Anspruch 1, dadurch gekennzeichnet, dass man als Metalloxide Ca- oder Mg-oxid einzeln oder in Mischung verwendet.

5. Verfahren nach Anspruch 1, dadurch gekennzeichnet, dass man als Metallhydroxide Aluminiumhydroxid, Alkali- oder Erdalkalimetallhydroxide einzeln oder in Mischung verwendet.

6. Verfahren nach Anspruch 1, dadurch gekennzeichnet, dass man als Metallhydroxide K-, Na-, Mg-, Ca-Hydroxide einzeln oder in Mischung verwendet.

7. Verfahren nach Anspruch 6, dadurch gekennzeichnet, dass man als Metallhydroxid Natronkalk verwendet.

8. Verfahren nach Anspruch 1, dadurch gekennzeichnet, dass man bei Temperaturen von 400-460°C arbeitet.

9. Verfahren nach Anspruch 1, dadurch gekennzeichnet, dass man bei Drucken von über 0,7 bar arbeitet.

10. Verfahren nach Anspruch 1, dadurch gekennzeichnet, dass man bei Atmosphärendruck arbeitet.

**Claims**

1. A process for the preparation of perylene by heating a perylenecarboxylic acid or an anhydride or salt thereof, in the presence or absence of a metal oxide, metal hydroxide or metal carbonate, to temperatures in the range from 380° to 550°C, which process is carried out under normal or slightly reduced pressure and the perylene so obtained is removed from the reaction vessel with a stream of inert gas.

2. A process according to claim 1, wherein the starting material is perylenetetracarboxylic dianhydride.

3. A process according to claim 1, wherein the metal oxide is aluminium oxide, zinc oxide, an alkali metal oxide or alkaline earth metal oxide, individually or in admixture.

4. A process according to claim 1, wherein the metal oxide is calcium or magnesium oxide, individually or in admixture.

5. A process according to claim 1, wherein the metal hydroxide is aluminium hydroxide or an alkali metal hydroxide or alkaline earth metal hydroxide, individually or in admixture.

6. A process according to claim 1, wherein the metal hydroxide is potassium hydroxide, sodium hydroxide, magnesium hydroxide or calcium hydroxide, individually or in admixture.

7. A process according to claim 6, wherein the metal hydroxide is soda lime.

8. A process according to claim 1, wherein the reaction temperature is in the range from 400° to 460°C.

9. A process according to claim 1, wherein the reaction is carried out under a pressure above 0.7 bar.

10. A process according to claim 1, wherein the reaction is carried out under atmospheric pressure.

## Revendications

1. Procédé de préparation du pérylène par chauffage d'acides pérylène-carboxyliques, de leurs anhydrides ou de leurs sels, en présence ou non d'oxydes, d'hydroxydes ou de carbonates de métaux, à des températures comprises entre 380 et 550°C, procédé caractérisé en ce qu'on opère sous la pression normale ou sous une pression légèrement réduite et en ce qu'on élimine le pérylène formé du récipient réactionnel par un courant de gaz inerte.

2. Procédé selon la revendication 1 caractérisé en ce qu'on part du dianhydride de l'acide pérylène-tétracarboxylique.

3. Procédé selon la revendication 1 caractérisé en ce qu'on utilise, comme oxydes de métaux, l'oxyde d'aluminium, l'oxyde de zinc, des oxydes de métaux alcalins ou des oxydes de métaux alcalino-terreux, isolément ou en mélange.

4. Procédé selon la revendication 1 caractérisé en ce qu'on utilise, comme oxydes de métaux, l'oxyde de calcium ou l'oxyde de magnésium, isolément ou en mélange.

5. Procédé selon la revendication 1 caractérisé en ce qu'on utilise, comme hydroxydes de métaux, l'hydroxyde d'aluminium, des hydroxydes de métaux alcalins ou des hydroxydes de métaux alcalino-terreux, isolément ou en mélange.

6. Procédé selon la revendication 1 caractérisé en ce qu'on utilise, comme hydroxydes de métaux, les hydroxydes de K, de Na, de Mg ou de Ca, isolément ou en mélange.

7. Procédé selon la revendication 6 caractérisé en ce qu'on utilise, comme hydroxydes de métaux, la chaux sodée.

8. Procédé selon la revendication 1 caractérisé en ce qu'on opère à des températures de 400 à 460°C.

9. Procédé selon la revendication 1 caractérisé en ce qu'on opère sous des pressions supérieures à 0,7 bar.

10. Procédé selon la revendication 1 caractérisé en ce qu'on opère sous la pression atmosphérique.